# EUROPEAN PATENT APPLICATION

(11) **EP 1 847 598 A1**
(43) Date of publication of application: **24.10.2007**
(21) Application number: 05784933.3
(22) Date of filing: 08.09.2005
(51) Int. Cl.: C12N 9/10

(54) **GTF CELLS, VECTORS AND SEQUENCES AND APPLICATIONS THEREOF IN THE FOOD SECTOR**

(30) Priority: 11.09.2004 ES 200402175
(71) Applicant: Consejo Superior de Investigaciones Cientificas, 28006 Madrid (ES); Universidad De Cantabria, 39005 Santander (ES); UNIVERSIDAD DEL PAIS VASCO, 48940 Leioa (Vizcaya) (ES)
(72) Inventor: Lopez Garcia, P., Centro de Investigaciones Biol., 28040 Madrid (ES); Werning, MarÍa, L., Centro Investigaciones Biol., 28040 Madrid (ES); Irastorza Iribas, Ana, E-48940 LEIOA (ES); Duenas Chasco, MarÍa Teresa, E-48940 LEIOA (ES); Ibarburu Lopez, Idoia, 48940 LEIOA (ES); Navas Mendez, Jesús, 39005 SANTANDER (ES)
(74) Representative: Ungria Lopez, Javier
(86) International application number: PCT/ES2005/070127
(87) International publication number: WO 2006/032718

(57) **Abstract**

The invention relates to GTF micro-organisms, vectors and sequences which are formed by or comprise gene *gtf* de *P.damnosus* 2.6 which encodes a glycosyltransferase that is located in the membrane and which enables the production of exopolysaccharides of the -glucan type by overexpression of said recombinant gene. The fact that only one gene is involved in the synthesis of the aforementioned enzyme facilitates the optimisation of the expression conditions which enable the production of -glucans on an industrial scale. Said polymers, which can be used as additives in numerous foods, also have properties that are beneficial to human health. Moreover, said transformed GTF micro-organisms can be used in processes for the fermentation of foods, such as dairy products, alcoholic drinks and oats.

## Description

### SECTOR OF THE ART

Biotechnology for the food sector. More specifically, fermentation of foods and drinks and in particular the obtaining of micro-organisms that produce exopolysaccharides (-glucans) and food additives.

### STATE OF THE ART

Exopolysaccharides (EPSs) secreted by various bacteria play an important role as adherence factors in the interactions of bacteria with plants as signalling molecules or as protector agents towards situations of stress or infections by phages (van Kranenburg et al. (1999) Curr. Opin. Biotech. 10:498-504). EPSs are produced by both Gram-negative and Gram-positive bacteria. Some of these polymers possess unique properties that make them suitable for being used as stabilising agents, thickeners, gelling agents and emulsifiers. They are used as additives in the manufacture of numerous foods such as dairy derivatives (yoghurts, shakes and ice-creams), fruit juices and cereal-based preparations. Among the most representative EPSs produced by Gram-negative bacteria we can mention xanthan, produced by *Xanthomonas campestris* and used in cosmetics in rubber form as a thickener, stabiliser and suspension agent (Becker et al. (1998) Appl. Microbiol. Biotechnol. 50: 145-152). Also acetan, produced by *Acetobacter xylinum* is used for the production of substitutes for cream and vinegar (Sutherland (1998) Trends Biotechnol. 16: 41-46). Moreover, capsular polysaccharides produced by *Sphingomonas,* which possess viscous properties, are used as stabilising agents for foods (Sutherland (1998) Trends Biotechnol. 16: 41-46).

Gram-positive bacteria and specifically lactic acid bacteria (LAB) produce various EPSs of industrial interest. A clear example is dextrans produced by *Leuconostoc mesenteroides,* used in the synthesis of matrices employed in chromatography techniques for coating metal surfaces or for stabilising syrups. Moreover, some strains of LAB belonging to the genera Streptococcus, Lactobacillus and Lactococcus produce EPSs which are being used in situ for improving the texture of fermented products such as yoghurt and cheese (de Vos (1999) Cur. Opin. Biotechn. 10: 483-484). Nevertheless, the large scale production of EPSs from LAB is currently restricted owing to the low levels produced (40-800 mg l⁻¹ of culture) in comparison with *Xanthomonas campestris* which can produce from 10 to 25 g 1-¹ or convert 60 to 70% of substrate sugar into xanthan by continuous culture.

Moreover, studies have been conducted which indicate that the EPSs produced by LAB possess beneficial effects for human health as stimulators of the immune system and it has therefore been proposed to use the producer strains in order to obtain functional foods (Jolly et al. (2002) Antonie van Leeuwenhoek 82: 367-374). Moreover, some of the EPSs are -glucans and there exists evidence that these homopolysaccharides contribute to reducing the levels of serum cholesterol (Behall et al. (1997) J. Am. College Nutr. 16: 64-51) Hence the interest in incorporating -glucans into foods and dietary products and the need to have efficient methods for their large scale production.

The structural characterisation of EPSs produced by strains isolated from cider *P*. *damnosus 2.6* (Duenas et al. Carbohidr. Res. 303: 453-458), *Lactobacillus* sp. G77 (Dueñas et al. Carbohidr. Res. 307: 125-133) and *Oenococcus oeni* 14 (results not published) revealed that they all synthesised the same homopolysaccharide of the type -D-glucan. It has also been demonstrated that both *P. damnosus* 2.6 and *Lactobacillus* sp. G77 can grow and produce EPS during the production of fermented foods based on oats, improving their organoleptic qualities (Olof Mårtensson et al. (2002) Nutrition Research 22: 1461-1473).

A high proportion of the world adult population currently displays intolerance to lactose and a social awareness is being created around the allergic reactions produced by milk and soy. Oat is a cereal which possesses a high nutritional value owing to its content in proteins, vitamin E, unsaturated fatty acids and essential minerals. It also contains 4-6% of -glucans, which reduce the level of serum cholesterol in human beings. This effect has been noted in clinical studies on the ingestion of oat-based foods (see details at http//www.adavena.com) and in fact the FDA (Food and Drug Administration, USA) has authorised the labelling "product beneficial for health" in food preparations based on oat containing 0.75 g of soluble fibre ( - glucan). The company Cereal Base (CEBA) (Lund, Sweden) specialises in the production of foods based on cereals as an alternative to dairy products. It has developed a technology known as Adavena which has permitted an oatmilk to be obtained with which products can be prepared as substitutes for yoghurts, dairy desserts, ice-creams, etc. fermented with LAB (Mårtensson et al., 2002). The nutritional and dietary properties of these preparations are improved if the content of -glucan in them is increased, by incorporating the producer bacteria or by the direct addition of these polymers.

Nevertheless, the genes or metabolic pathways involved in this production of -glucans in micro-organisms of potential usefulness in the food sector are not known.

### DESCRIPTION

### Brief description

An object of the present invention consists of a DNA sequence, hereinafter gtf DNA sequence of the present invention, coming from GRAS (Generally Recognised as Safe) lactic acid bacteria and encoding a protein with glycosyltransferase activity which permits the production of exopolysaccharides (EPSs) and which consists of one of the nucleotide sequences selected from between:
a) the nucleotide sequence identified as SEQ ID N09 or a fragment thereof; and
b) a nucleotide sequence analogous to the sequence defined in a).

The invention also refers to a GTF expression vector which comprises the GTF DNA sequence of the invention, hereinafter the GTF expression vector of the invention, and which permits the transformation of micro-organisms or cells and the subsequent obtaining of micro-organisms capable of expressing the GTF protein (SEQ ID NO6) and which permits the host to acquire the capacity, or improve the already existing capacity, to produce EPS.

Another object of the present invention is the use of the GTF DNA sequence or the GTF expression vector in a cell transformation process in order to obtain GTF cells.

A further object of the invention consists of a protein with glycosyltransferase activity which consists of one of the sequences of amino-acids selected from between:
a) the amino acid sequence identified as SEQ ID N06 or a fragment thereof; and
b) an amino acid sequence analogous to the sequence defined in a).

Another object of the invention consists of a cell, hereinafter the GTF cell of the present invention, which comprises a sequence of GTF DNA or a pGTF expression vector of the invention, capable of expressing in recombinant form the GTF protein (SEQ ID NO6) and which therefore acquires the capacity, or improves the already existing capacity, to produce EPS.

Another object of the present invention is the use of the GTF cells for producing EPSs by means of a procedure comprising:
a) the growth of the GTF cell in a suitable medium and under conditions which permit expression of GTF expression vector or of GTF DNA sequence,
b) the production of EPSs by the GTF cell of a), and
c) purification of the EPSs of b)
   Finally, a further object of the invention consists of the use of the GTF cells in fermentation processes for foods, among others and by way of illustration without limiting the scope of this invention, those belonging to the following group: dairy products, alcoholic drinks and oats.

### Detailed description

The invention tackles the problem of providing new genetic and biological tools permitting the expression of new enzymes useful for the production of EPSs and the generation of micro-organisms of industrial value.

The solution proposed by this invention is based on the fact that the inventors have identified the gene gtf coding an enzyme with glycosyltransferase (GTF) activity, starting from genome material of *Pediococcus damnosus 2.6.* The recombinant expression of this gene in *streptococcus pneumoniae* and in *Lactococcus lactis* demonstrated the glycosyltransferase activity of the enzyme encoded by that gene, along with the possibility of using that gene to successfully transform Gram-positive bacteria, including lactic acid bacteria, which acquire the capacity of its expression. The strains of lactic bacteria can be useful for obtaining EPSs for their later use as additives in the food sector and, moreover, their later genetic manipulation in order to confer GRAS quality on them which permits their use in the transformation of foods with new properties. The overproduction of GTF with glycosyltransferase activity could permit those polymers to be obtained on the large scale for their incorporation as additives in foods and dietary preparations which possess beneficial properties for human health.

The comparison of the sequence of that gtf gene with those deposited in databases showed the absence of significant homologies. Nevertheless, when comparing the sequence of the GTF protein product of the gtf gene it was observed that it is homologous with glycosyltransferases of COG1215 family. Moreover, the GTF protein presents a relatively high (34%) homology with the Tts protein of *Streptococcus pneumoniae,* a glycosyltransferase which is the only enzyme required for the biosynthesis and secretion of -D-glucan similar to that of P. *damnosus* (Llul et al. (1999) J. Biol. Chem. 24: 21053-21061). This characteristic contrasts with the operon complexes (consisting of ten genes) which encode proteins required for synthesis and secretion of bacterial heteropolysaccharides and homopolysaccharides currently used at the industrial level (Jolly and Stingele (2001) Int. Dairy J. 11: 733-745 ) and represents a clear and important technical advantage. As a consequence, a protein with similar characteristics to Tts, but coming from a non-pathogenic micro-organism having food quality would have potential for the production of -D-glucans by genetic manipulation and would be of industrial interest.

The same research team has observed that, by means of the PCR characterisation of the gene *gtf* in different bacterial strains producing -D-glucan, of homo- or hetero-polysaccharides, the protein GTF is specifically implicated in the biosynthesis of homopolysaccharides consisting of -D-glucan and no other type of homo- and hetero-polysaccharides (see Spanish patent application "PROCESS FOR MOLECULAR DETECTION OF LACTIC ACID BACTERIA PRODUCERS OF -GLUCANS" (2004)).

So, an object of the present invention consists of a DNA sequence, hereinafter GTF DNA sequence of the present invention, coming from GRAS lactic acid bacteria and encoding a protein with glycosyltransferase activity which permits the production of exopolysaccharides and which consists of one of the nucleotide sequences selected from between:
a) the nucleotide sequence identified as SEQ ID NO9 or a fragment thereof; and
b) a nucleotide sequence analogous to the sequence defined in a).

In the sense used in this description, the term "analogous" intends to include any DNA sequence that can be isolated or constructed on the basis of the sequence of nucleotides shown in SEQ. ID NO9, for example, by means of introducing conservative or non-conservative substitutions of nucleotides, including the insertion of one or more nucleotides, the addition or one or more nucleotides at either end of the molecule or the deletion of one of more nucleotides at either end or within the interior of the sequence.

In general, an analogous DNA sequence is substantially homologous with the sequence of nucleotides identified as SEQ ID NO9. In the sense used in this description, the expression "substantially homologous" signifies that the nucleotide sequences in question have a degree of identity at the nucleotide level of at least 60%, preferably at least 85%, and more preferably at least 95%.

The GTF DNA molecule of the invention comes from P. *damnosus* 2.6 and can be found in similar forms in other micro-organisms, among others, by way of illustration and without limiting the scope of the invention: *Lactobacillus* sp. and *Oenococcus oeni,* where they can be natural or they could otherwise be the result of a gene transformation process in which the transformed organism reproduces those DNA molecules. The DNA sequence of the invention can be isolated by means of conventional techniques starting from the DNA of any micro-organism containing it, by means of using probes or oligonucleotides, prepared thanks to the information of the nucleotide sequence of that DNA molecule, provided in this invention by an expert in the subject. Results obtained by the same research team indicate that the production of -glucan by the strains *Pediococcus damnosus* 2.6, *Lactobacillus* sp G77 and *Oenococcus oeni* 077 require the expression of *gtf* gene, whose allelic forms are virtually identical in all strains, though their genome location and the elements regulating their expression are different in each strain (see patent application "PROCESS FOR MOLECULAR DETECTION OF LACTIC ACID BACTERIA PRODUCERS OF -GLUCANS" (2004)).

Moreover, starting from the GTF DNA sequence of the present invention, an expert in the subject can obtain other deletions or protein fragments derived from this GTF protein or from its homologous forms which maintain its glycosyltransferase activity. Therefore, the DNA molecule of the invention includes fragments of it which present said glycosyltransferase activity.

In a particular embodiment, the GTF DNA sequence of the invention is a DNA molecule of P. *damnosus* 2.6 and in particular SEQ ID NO9.

The GTF DNA sequence of the invention can in general be used in the generation of an expression vector which permits expression of these proteins with glycosyltransferase activity in a broad range of host cells.

Therefore, the invention also refers to a GTF expression vector which comprises the GTF DNA sequence of the invention, hereinafter the GTF expression vector of the invention, and which permits the transformation of micro-organisms or cells and the subsequent obtaining of micro-organisms capable of expressing the GTF protein (SEQ ID NO6) and which permits the host to acquire the capacity, or improve the already existing capacity, to produce EPS.

In general, the expression vector of the present invention comprises at least one GTF DNA sequence of the invention and at least one promoter which directs the transcription of the gene of interest, to which it is operatively linked, and other sequences necessary or appropriate for the transcription of the gene of interest and its regulation in time and place, for example, starting and finishing signals, cutting sites, polyadenylation signal, replication origin, transcriptional activities, transcriptional repressors, insertion sequences, etc. Examples of suitable expression vectors can be selected according to the conditions and needs of each specific case from among plasmids and cosmids with a bacterial replication origin so that it can be amplified in multicopy starting from the extra chromosomal elements in bacteria or integrative vectors so that it can be amplified in monocopy starting from the chromosome in bacteria, along with a marker that can be used for selecting the transformed cells different from the gene of interest. The choice of vector will depend on the host cell into which it is going to be introduced later on. By way of example, the vector where said DNA sequence is introduced can be a plasmid which, when introduced in a host cell, becomes integrated into the genome of said cell and is replicated along with the chromosome of the host cell.

The vector of the invention can be obtained by conventional methods known to technicians in the field (Sambrook et al. (1989) Molecular cloning: a laboratory manual. Cold Sprig Harbour Laboratory Press N.Y.). A particular object of the present invention consists of the plasmid pGTF which contains the GTF DNA sequence of the present invention (see Example 2).

Another object of the present invention is the use of the GTF DNA sequence of the GTF expression vector in a cell transformation process in order to obtain GTF cells.

A further object of the invention consists of a protein with glycosyltransferase activity which consists of one of the sequences of amino-acids selected from between:
a) the sequence of amino-acids identified as SEQ ID NO6 or a fragment thereof; and
b) a sequence of amino-acids analogous to the sequence defined in a).

Another object of the invention consists of a cell, hereinafter the GTF cell of the present invention, which comprises a sequence of GTF DNA or a pGTF expression vector of the invention, capable of expressing in recombinant form the GTF protein (SEQ ID NO6) and which therefore acquires the capacity, or improves the already existing capacity, to produce EPS.

The host cells that can be transformed with that expression vector can be both Gram-positive and Gram-negative bacterial cells, by way of illustration and without limiting the scope of the present invention, LABs belonging to the genera *Lactococcus, Lactobacillus* and Streptococcus such as S. *thermophilus* various subspecies of *L*. *lactis* and *Lactobacillus delbrueckii* subsp. bulgaricus, which are currently used in the production of dairy products.

A particular embodiment of the present invention consists of the GTF cell *L*. *lactis* MG1363[pGTF] and the cell *S. pneumoniae* R61[pGTF].

The GTF cells of the invention can be used for the overproduction of EPSs for their subsequent use in the different possible applications. So, another object of the present invention is the use of the GTF cells for producing EPSs by means of a procedure comprising:
a) growth of the GTF cell in a suitable medium and under conditions which permit expression of GTF expression vector or of GTF DNA sequence,
b) the production of EPSs by GTF cell of a), and
c) purification of the EPSs of b)
   Purification of the EPSs produced by GTF cells can be carried out by conventional techniques known to an expert in the subject.

As stated earlier, the EPSs thus obtained can be used as stabilising agents, thickeners, gelling agents and emulsifiers in many different processes of handling, processing and transformation of foods, such as for example as additives in the manufacture of numerous foods such as dairy derivatives (yoghurts, shakes and ice-creams), fruit juices and cereal-based preparations, as substitutes for cream and vinegar (Sutherland (1998) Trends Biotechnol. 16: 41-46), as stabilising agents for foods (Sutherland (1998) Trends Biotechnol. 16: 41-46), in cosmetics in rubber form as a thickener, stabiliser and suspension agent (Becker et al. (1998) Appl. Microbiol. Biotechnol. 50: 145-152) and also in the synthesis of matrices employed in chromatography techniques for coating metal surfaces or for stabilising syrups.

On the other hand, these results enable new possibilities to be opened up for transforming a GRAS bacterial system, in other words, micro-organisms among others, and by way of example without limiting the scope of the present invention, strains of LAB belonging to genera of the following group: *Lactobacillus, Leuconostoc, Pediococcus and Oenococcus -* which can be used in the food industry in fermentation processes for foods, among others, and by way of example without limiting the scope of the present invention, those belonging to the following group: dairy products, alcoholic drinks and oats, which permit improvement of the texture of the fermented products. e.g., yoghurt and cheese (de Vos (1999) Cur. Opin. Biotechn. 10: 483-484). or the improvement of their organoleptic qualities (Olof Mårtensson et al. (2002) Nutrition Research 22: 1461-1473).

Finally, a further object of the invention consists of the use of the GTF cells in fermentation process for foods, among others and by way of illustration without limiting the scope of this invention, those belonging to the following group: dairy products, alcoholic drinks and oats.

### DESCRIPTION OF THE FIGURES

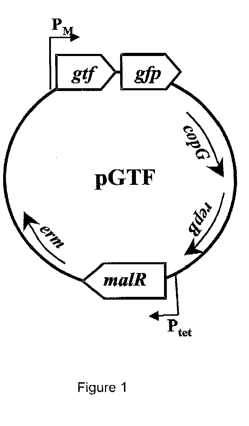

**Figure 1.- Physical map of pGTF plasmid.** The genetic symbols used are: *gtf,* encodes the glycosyltransferase; *gfp* encodes the green fluorescent protein; *erm* encodes the protein responsible for erythromycin resistance; *malR* encodes the transcriptional repressor MalR; P_{M}, promoter of *gtf* and *gfp, copG* and *repB* encode the proteins implicated in plasmid replication.

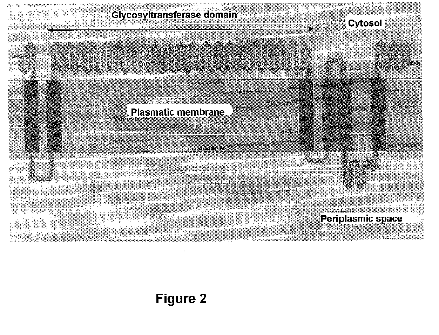

**Figure 2.- Prediction of the secondary structure of GTF protein from P. *damnosus* 2.6 using the SOSUI program.** The colours of the amino-acids correspond to: black, hydrophobic; blue, with polar chain; blue and black, positively charged; red; negatively charged.

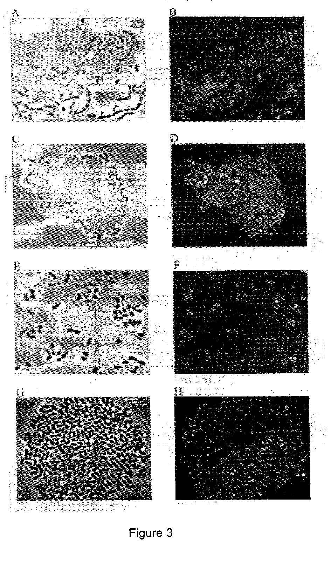

**Figure 3.- Detection in *S. pneumoniae* and in *L. lactis* of gtf and *gfp* expression respectively by microscopic detection of agglutination and by fluorescence microscopy.** Contains photographs of cell cultures of *S. pneumoniae* R61[pLS1RGFP] (A and B) and R61[pGTF] (C and D) and of *L*. *lactis:* MG1363[pLS1RGFP] and MG1363[pGTF] detected by phase contrast microscopy (A, C, E and G) and by fluorescence microscopy (B, D, F and H).

### EXAMPLES OF EMBODIMENT

### Example 1: Cloning and characterisation of gtf gene from P. damnosus 2.6.

The cloning of the 5' region of the gene was carried out by PCR amplification using the total DNA of the plasmids carried by *P. damnosus* 2.6 and the degenerate oligonucleotides 5'-TAYGAYAAYACNCARGARGT-3' (Oligo I, SEQ ID NO1) and 5'-ACRAARTARTCRTARTCRTG-3' (Oligo II, SEQ ID NO2) (Y = T or C; W; R = A or G; N = A, C, G or T). These oligonucleotides were designed on the basis of the amino-acid sequence conserved from the glycosyl transferase of *P. damnosus* IOEB8801 (Walling et al. (2001) Lait 81: 289-300) since the nucleotide sequence of the coding gene has not been published, nor deposited in the DNA sequence data banks. The amplified fragment of 1.8 kb was cloned in the vector pCR 2.1-TOPO (Invitrogen) and the recombinant plasmid obtained was established in Escherichia coli DH5 . The determination of the nucleotide sequence of the DNA fragment revealed the existence of an open reading frame lacking its amino terminal region. The DNA sequence obtained was used for synthesising the oligonucleotides 5'-ACGCCCTGCGTGTTATCATA-3' (SEQ ID NO3) and 5'-TGTGTAATGGCACTCACGAC-3' (SEQ ID NO4) and by means of reverse PCR reaction the 5' end of the *gft* gene was amplified, which was cloned using the same vector, method and host bacterium as were used for cloning the 3' end of the gene. The sequence of 3352 nucleotides of the cloned regions is shown in SEQ ID NO5 (nucleotides 1-621 of the 3' region of the *mobA* gene, nucleotides 819-1085 of the hypothetical ORF1 gene and nucleotides 1282-2982 of the *gtf* gene -CDS region). The *gft* gene (SEQ ID NO 9) encodes the GTF protein of 567 amino acids detailed in SEQ ID NO6.

The comparison of the DNA sequence of *gtf* gene from *P. damnosus* with those deposited in databases shows the absence of significant homologies. Nevertheless, when comparing the protein sequence (with the Swissprot database) encoded by the gtf gene (GTF) it was observed that it is homologous to the glycosyltransferases of the COG1215 family. Also, the GTF protein presents a relatively high (34%) homology with the Tts protein of *Streptococcus pneumoniae,* a glycosyltransferase which is the only enzyme required for the biosynthesis and secretion of -D-glucan similar to that of *P. damnosus* (Llul et al. (1999) J. Biol. Chem. 24: 21053-21061). This feature contrasts with the operon complexes (consisting of ten genes) which encode proteins required for the synthesis and secretion of bacterial heteropolysaccharides and homopolysaccharides currently used at industrial level (Jolly and Stingele (2001) Int. Dairy J. 11: 733-745). As a consequence, a protein with features similar to Tts, but coming from a non-pathogenic micro-organism having food quality would have potential for the production of -D-glucans by genetic manipulation and would be of industrial interest.

Results obtained by the same research group indicate that production of -glucan by *Pediococcus damnosus* 2.6, *Lactobacillus* sp G77 and *Oenococcus oeni* 077 strains, require the expression of the gtf gene, whose allelic forms are virtually identical in all strains, though their genomic location and elements regulating their expression are different in each strain (see patent application "PROCESS FOR MOLECULAR DETECTION OF LACTIC ACID BACTERIA PRODUCERS OF -GLUCANS" (2004)).

### Example 2: Construction of the pGTF vector which permits overexpression of GTF protein of Pediococcus damnosus 2.6.

As already mentioned earlier, the homology of GTF with Tts glycosyltransferase of *S*. *pneumoniae* (Llul et al. (1999) J. Biol. Chem. 24: 21053-21061) and which constitutes the capsule of the serotype 37 (Llul et al. (1999) J. Biol. Chem. 24: 21053-21061) indicated that GTF is possibly the only protein responsible for biosynthesis of the exopolysaccharide of *P. damnosus.* For this reason, as part of this invention, the recombinant plasmid pGTF overproducer of GTF and which contains the DNA sequence of the gene *gft* of the invention (SEQ ID NO9) was constructed (Figure 1). In the expression vector pLS1RGFP (Nieto et al. (2000) Plasmid 43: 205-213) between the inducible promoter P_{M} and the *gfp* reporter gene a fragment of DNA was cloned which includes the coding region of the gtf gene. To do this, the region which includes the gtf gene was amplified using Pfu DNA polymerase (Stratagene) and using as substrate the total DNA of the plasmids carried by *P*. *damnosus* 2.6 and the oligonucleotides 5'-TCTAGAAATTAAAGGAATGTGTAA-3' (SEQ ID NO7) and 5'-TCTAGATTAATCATTCCAATCAACTG-3' (SEQ ID NO8), which include the recognition sequence for restriction enzyme Xbal. The amplified fragment of 1.734 kb was cloned in the correct orientation with respect to P_{M} promoter in the Xbal unique site of pLS1RGFP and, subsequently, the recombinant plasmid was established in the non-capsulated strain R61 of *S. pneumoniae* (strain R6 whose genome has been sequenced by Hoskins et al. (2001) J. Bacteriol. 183: 5709-5717 and which was named R61 by Dr Sanford Lacks in: Lacks (1968) Genetics 60: 685-706) by transformation and selection of transformants due to resistance to 5 g ml⁻¹ of erythromycin (resistance marker of the plasmid vector) as described in Lacks (1968) Genetics 60: 685-706), obtaining the strain S. *pneumoniae* R61[pGTF]. The pGTF plasmid, which is capable of replicating in Gram-positive and Gram-negative bacteria, contains a transcriptional fusion P_{M}*-gtf-gfp,* which permits an overexpression by growth of the carrier cells in medium containing maltose and a detection of the expression from the promoter P_{M} by measurement of GFP protein fluorescence (Nieto et al. (2000) Plasmid 43: 205-213).

### Example 3.- In vitro detection of GTF function integrated in S. pneumoniae membranes

Glycosyltransferase Tts of S. *pneumoniae* is an integral membrane protein (Llul et al. (1999) J. Biol. Chem. 24: 21053-21061). The analysis of the prediction of topological location of GTF from *P. damnosus 2.6* using the SOSUI program (http://sosui.proteonem.bio.tuat.ac.ip/sosuiframe0.html) revealed that the catalytic domain of the protein (amino-acids 83 to 377) is located in the bacterial cytoplasm and that there exist six transmembrane regions: two located at the amino terminal end of the protein preceding the catalytic domain of it and four located in its carboxylic region (Figure 2). For the reasons mentioned earlier, membranes of *S. pneumoniae* R61 strains carriers of the pGTF plasmid or of the pLS1RGFP plasmid vector were purified and the glycosyltransferase activity was determined using UDP-glucose as substrate. To do this, cells of the strains of *S. pneumoniae* R61 [pLS1RGFP] (Nieto et al. (2000) Plasmid 43: 205-213) and R61[pGTF] were grown in 1 1 of Tood-Hewitt medium (Difco) supplemented with 0.5% yeast extract, 0.1% saccharose and 0.8% maltose to an A₆₅₀ of 0.4. The cultures of both strains showed a similar duplication time (Table 1), indicating that expression of the P_{M}-*gtf-gfp* fusion did not have any deleterious effect on *S*. *pneumoniae.* The assessment of the expression from P_{M} promoter was carried out by means of fluorescence determination due to the GFP protein of 0.1 ml of the cultures by means of fluorescence spectroscopy (Nieto et al. (2000) Plasmid 43: 205-213). The results obtained (Table 1) showed that in both strains analysed there existed GFP protein expression that was slightly less in R61[pGTF] than in the strain carrying the vector, an effect that was observed in constructions prior to inserting a gene between the P_{M} promoter and the *gfp* gene (results not shown) .

In order to obtain the membranes required for determining glycosyltransferase activity, the cells present in 1 litre of medium were sedimented by centrifugation at 12000 x g for 20 minutes (min) at 4°C and resuspended in buffer A (70 mM Tri.HCl pH 7.0, 9 mM MgCl₂, 1 mM CaCl₂) with 0.2 M phenylmethylsulphonyl fluoride (PMSF) and sedimented by centrifugation at 10000 x g for 10 min at 4°C. The bacteria resuspended in buffer A were subjected to mechanical rupture by passing them twice through a French press (Aminco). The homogenised product was centrifuged at 12000 x g for 15 min at 4°C and the supernatant was again centrifuged at 12000 x g for 30 min at 4°C. Sedimented membranes were once more resuspended in 2 ml of buffer A containing 0.2 mM of PMSF and stored at -80°C. Protein concentration of the membrane preparations was determined using the BCA Protein Assay system (Pierce). Determination of the glycosyltransferase activity was done using 30 M (0.1 Ci) UDP-[¹⁴C]glucose (specific activity of 333 mCi/mmol) in a buffer A supplemented with 50 mM NaCI in a total volume of 100 1 and membranes in a range of protein concentrations between 0.05 and 0.5 mg ml⁻¹. The reactions were conducted by incubation at 30°C for 15 min. The reaction was terminated by addition of SDS to a final concentration of 0.5% and incubation at 37°C for 15 min. Bovine seroalbumin was then added to a final concentration of 0.4% along with 1 ml of 10% trichloroacetic acid (TCA). Following incubation for 30 min at 0°C, the mixtures were filtered through Wathman GF/A filters and were washed with 15 ml of 10% TCA. The filters were dried at 65°C for 20 min and the radioactivity present in them was measured in a scintillation counter (LKB Wallack). The results obtained (Table 1) revealed the existence of glycosyltransferase activity in the membranes of the two strains analysed. The levels were approximately 2.5 times higher in strain R61[pGTF] in comparison with the control strain. This increase was presumably due to the glycosyltransferase activity of the GTF from P. *damnosus.* In the case of control strain R61 [pLS1RGFP] the levels of activity detected could be due to the products of chromosomal genes cpoA and *epsG* of *S*. *pneumonias,* whose function is unknown, but by homology they are proposed as glycosyltransferases in the KEGG data bank (*http:*// *www. genome.jp*/*kegg*/)*.*

**Table 1. Effect: of the induction of the fusion P_{M}-gtf-gfp on the growth, GFP levels and glycosyltransferase activity in S. pneumoniae**

| **Strain** | **¹ Duplication time (min)** | **² GFP fluorescence (arbitrary units)** | **³ glycosyl-transferase activity (units)** |
|---|---|---|---|
| R61 [pLS1RGFP] | 30 | 152.85 | 243.71 ± 42 |
| R61 [pGTF] | 35 | 98.7 | 615.46 ± 193 |

| | | | |
|---|---|---|---|
| ¹ The duplication time of the bacteria was calculated using the measurement of A₆₅₀ during growth. ² The fluorescence of 0.1 ml of the cultures previously sedimented and resuspended in 0.1 ml of PBS buffer pH 7.2 was measured on micro-titration plates in an LS_50B spectrophotometer (Perkin Elmer) by means of excitation at a wavelength of 488 nm with an aperture of 5 and the detection of fluorescence was done at a wavelength of 511 nm with an aperture of 5. ³ Values represent the mean and standard deviation of 10 determinations using different membrane concentrations in the range 0.05 to 0.5 mg ml⁻¹ of protein. One unit of glycosyltransferase activity corresponds to the quantity of enzyme which catalyses the incorporation of a macromolar product of 1 pmol of glucose per mg of protein and per minute. | | | |

### Example 4.- Detection of GTF function in vivo in S. pneumoniae and L. lactis

The results of detection of glycosyltransferase activity indicated that pGTF plasmid encodes the active GTF protein. Since antibodies developed against the capsule of serotype 37 of *S*. *pneumoniae* are very specific and do not react with non-capsulated strains or with other serotypes of *S.* pneumoniae or with other bacteria, these antibodies were used in order to make an in vivo assessment of the functional expression of GTF from *Pediococcus* both in *S. pneumoniae* R61[pGTP] and in the strain MG1363 of *Lactococcus lactis* (to which the plasmid pGTF had been transferred by transformation). Functional expression of GTF was determined by means of the agglutination technique using antibodies developed against the capsule of the serotype 37 of S. *pneumoniae* and by microscopy (Llul et al. (1999) J. Biol. Chem. 24: 21053-21061). Cells from *S. pneumoniae* strains R61[pLS1RGFP] and R61[pGTF] were grown in Tood-Hewitt medium (Difco) supplemented with 0.5% yeast extract, 0.1% saccharose and 0.8% maltose to an A₆₅₀ of 0.4. Cells from the *L. lactis* strains MG1363[pLS1RGFP] and MG1363[pGTF] were grown in Tood-Hewitt medium (Difco) supplemented with 0.5% yeast extract, and 5% maltose to an A₆₆₀ of 0.5. One ml of each of the cultures was sedimented by centrifugation and cells were resuspended in 100 1 of PBS buffer pH 8.0 (10 mM Na₂HPO₄, 1 mM KH₂PO₄, 140 mM NaCl, 3 mM KCl). Ten M of each of the cultures were mixed with 10 1 of antibody developed against pneumococcus serotype 37 (Statens Seruminstitut, Denmark) and kept at 4°C for 2 hours. The preparations were analysed by phase contrast and fluorescence microscopy using a Zeiss Axioplan microscope (Universal microscope) with excitation fluorescence filters Standard FITC set D480/30 and emission TBP 460/530/610. The results obtained appear in Figure 3. The fluorescence of GFP protein permitted the detection by fluorescence microscopy of the existence of gene expression starting from the P_{M} promoter of pLS1RGFP and of pGTF in the cells of *S*. *pneumoniae* (Figures 3B and 3D) and L. lactis (Figures 3F and 3H) induced with maltose. The images obtained by fluorescence and phase contrast microscopy revealed that only the cells of *S. pneumoniae* (Figures 3C and 3D) and of *L*. *lactis* (Figures 3G and 3H) that were carriers of the pGTF plasmid and induced with maltose were capable of agglutination. These results show that pGTF confers *S*. *pneumoniae* and *L. lactis* with the capacity to synthesise the exopolysaccharide.

## Claims

1. GTF DNA sequence **characterised in that** it comes from GRAS lactic acid bacteria, it encodes a protein with glycosyltransferase activity which permits the production of exopolysaccharides (EPSs) and **in that** it consists of one of the nucleotide sequences selected from between:
a) the sequence of nucleotides identified as SEQ ID N09 or a fragment thereof; and
b) a sequence of nucleotides analogous to the sequence defined in a).

2. GTF DNA sequence according to claim 1 **characterised in that** it is the SEQ ID NO9.

3. GTF expression vector **characterised in that** it comprises the GTF DNA sequence according to claims 1 and 2.

4. GTF expression vector according to claim 3 **characterised in that** it is the pGTF plasmid.

5. Use of the GTF DNA sequence according to claims 1 and 2 or of the GTF expression vector according to claims 3 and 4 in a process of cell transformation.

6. Protein with glycosyltransferase activity **characterised in that** it consists of one of the sequences of amino-acids selected from between:
a) the sequence of amino acids identified as SEQ ID NO6 or a fragment thereof; and
b) a sequence of amino acids analogous to the sequence defined in a).

7. GTF cell **characterised in that** it comprises a GTF DNA sequence according to claims 1 and 2 or a pGTF expression vector according to claims 3 and 4, capable of expressing in recombinant form the GTF protein according to claim 6 and therefore acquires the capacity, or improves the already existing capacity, to produce EPSs.

8. Use of GTF cells according to claim 7 in a procedure for producing EPSs.

9. Use of GTF cells according to claim 8 **characterised in that** the procedure comprises:
a) the growth of the GTF cell in a suitable medium and under conditions which permit expression of the GTF expression vector or of the GTF DNA sequence,
b) the production of EPSs by the GTF cell, and
c) purification of the EPSs of b).

10. Use of the GTF cells according to claim 7 in food fermentation processes.

11. Use of the GTF cells according to claim 10 **characterised in that** the fermentation processes for foods belong to the following group: dairy products, alcoholic drinks and oats.


SEQUENCE LISTING

<110> CONSEJO SUPERIOR DE INVESTIGACIONES CIENTÍFICAS

<120> GTF CELLS. VECTORS AND SEQUENCES AND APPLICATIONS THEREOF IN
THE FOOD SECTOR

<130> Gene gtf

<160> 9

<170> PatentIn version 3.2

<210> 1
<211> 20
<212> DNA
<213> Artificial sequence

<220>
<223> Oligo degA

<220>
<221> misc_feature
<222> (12)..(12)
<223> n is a, c, g, or t

<400> 1
taygayaaya cncargargt 20

<210> 2
<211> 20
<212> DNA
<213> Artificial sequence

<220>
<223> Oligo degB

<220>
<221> misc_feature
<222> (12)..(12)
<223> n is a, c, g, or t

<400> 2
taygayaaya cncargargt 20

<210> 3
<211> 20
<212> DNA
<213> Artificial sequence

<220>
<223> Oligo III

<400> 3
acgccctgcg tgttatcata 20

<210>  4
<211>  20
<212>  DNA
<213>  Artificial sequence

<220>
<223>  Oligo IV

<400>  4
tgtgtaatgg cactcacgac                                                    20


<210>  5
<211>  3352
<212>  DNA
<213>  Pediococcus damnosus


<220>
<221>  CDS
<222>  (1282)..(2982)

<400>  5
tgacaacacg caggaagttt taagaagagc ctttcctaac ggtaatttta atgaattacc      60

aatgattaaa caggaacaag cctatacagc cgtgatgtac tatgatcctg ttttaaagcc     120

atgtcaggct gaaacaattg aacagtggca agcaaatcca ccacaggtgt tcggtccccc     180

agaacatcaa caaggactag cttatttatc ggggcagctt agcttagatc agttagaaaa     240

tcatcactta caacgggttt taaagcatga tggcactaaa caactctttt ttggcgaatg     300

caaagccgat ccgacgatta agaacagtca gatcgagaaa atccaaaagc agttaaaagg     360

gcaacaagcc aaggacgacc agtatagaaa agtaaatatt ggacattatc aaccgctaaa     420

ttacaagcca gttagtccaa gctaccactt aaagacggcc tttagtaacg caatcatgac     480

cgccctatat gcccgtgatg aagattacga acggcaaaaa caggcgcaag gtttaaagga     540

gactgagtgg gaaatgacga aaaagcaacg gcaacaccaa actcgaaacc ggcatgaaga     600

tgggggcatg cacttgtaat ctaaatgtaa aattaaaagt gcaccaacgt gctcatttaa     660

tggtacaatg aacccataat atagggaaag gagtattcac atgtgacaaa gaaacaggaa     720

tggtttagct tagctcaagc tagtctcaag cttgaacgag gtagtacgta cgtgagtgtt     780

tggttaagac ggcaccctaa cgagttgcca agtgaaatgc tcatggaaac gggcaaagtt     840

aaattaatat ctgaagatgg cattgaatgg attaaaaacc acataaaaaa agagggcgtc     900

ctcgtaagca gtgaagttgc taacggggat cagcacctgg aagttacgat tctaggtgtc     960

accctcctaa caatccattt tacccggcgg gttaggggaa agctagtgga tcctaattgt    1020

tcaaaaaaca gcgaaaagac aaacgacgcg atactgccgc aaaactcagc cattgttttc    1080

taaggattaa gtcttttgga ttttgtgtat ctatcaactc cttaaagcct tctgagtcct 1140

ataataaccc aaaagtgatc tataaaatgc tctacgtcga tttaccgttg accgatagtt 1200

gaatagctca cggttagcta aaatatcgtt aaatgaaaaa tggaatattt tatggaaaaa 1260

aattaaagga atgtagtata a atg tta aat gat aat gat tca gaa cta aaa 1311
Met Leu Asn Asp Asn Asp Ser Glu Leu Lys
1               5                   10

aaa ttt cac ttg ttt cat tct aaa cca gtc ttt gta cca gtt att tta 1359
Lys Phe His Leu Phe His Ser Lys Pro Val Phe Val Pro Val Ile Leu
                15                  20                  25

att att tgg ttg ttt att atg tgc tta tat gaa tat tta aca tac aca 1407
Ile Ile Trp Leu Phe Ile Met Cys Leu Tyr Glu Tyr Leu Thr Tyr Thr
            30                  35                  40

gat agc ata ctt cct att tta gct aaa aag caa cca cta gaa gta att 1455
Asp Ser Ile Leu Pro Ile Leu Ala Lys Lys Gln Pro Leu Glu Val Ile
        45                  50                  55

tta cct ata ttc aat caa tta ttt gtg gca ctt ttc ttt ttg ctt gga 1503
Leu Pro Ile Phe Asn Gln Leu Phe Val Ala Leu Phe Phe Leu Leu Gly
    60                  65                  70

ata act aat att att atc gct atc cgc tat gca atg att aaa gac aaa 1551
Ile Thr Asn Ile Ile Ile Ala Ile Arg Tyr Ala Met Ile Lys Asp Lys
75                  80                  85                  90

gca aaa gaa tcc gaa cta gca ata ctc gca aaa gaa acg cct gca gac 1599
Ala Lys Glu Ser Glu Leu Ala Ile Leu Ala Lys Glu Thr Pro Ala Asp
                95                  100                 105

tgg cac cct aaa gtt gag tta ttg tat acg acc tat aat gat ttt ata 1647
Trp His Pro Lys Val Glu Leu Leu Tyr Thr Thr Tyr Asn Asp Phe Ile
            110                 115                 120

cct tat gca cta gct caa tgt tta aaa cag aca tat gat aac acg cag 1695
Pro Tyr Ala Leu Ala Gln Cys Leu Lys Gln Thr Tyr Asp Asn Thr Gln
        125                 130                 135

ggc gtt att ttg gat aac tct aca gac ccc aaa tac atc aag atg att 1743
Gly Val Ile Leu Asp Asn Ser Thr Asp Pro Lys Tyr Ile Lys Met Ile
    140                 145                 150

gat gat ttt gtg ata gcc cat cct aat gta aag tta gtc aga gat tct 1791
Asp Asp Phe Val Ile Ala His Pro Asn Val Lys Leu Val Arg Asp Ser
155                 160                 165                 170

caa aac aag cat gct aaa gct gga aac tta aac aat tat ttg tgt aat 1839
Gln Asn Lys His Ala Lys Ala Gly Asn Leu Asn Asn Tyr Leu Cys Asn
                175                 180                 185

ggc act cat gac tac gat tac ttt gtt atc cta gat agc gat gaa tta 1887
Gly Thr His Asp Tyr Asp Tyr Phe Val Ile Leu Asp Ser Asp Glu Leu
            190                 195                 200

tta gaa aat aga ttt gta gaa aaa tgt tta aag atg ttt tat tac aat 1935
Leu Glu Asn Arg Phe Val Glu Lys Cys Leu Lys Met Phe Tyr Tyr Asn

        205                 210                 215

gat att ggc att ctt cag tgt aat cac att agt gga caa aac cac aat 1983
Asp Ile Gly Ile Leu Gln Cys Asn His Ile Ser Gly Gln Asn His Asn
    220                 225                 230

tcg ttt atg cgt act ttc tct agt tct ggc aat att ttt tgg cca gtg 2031
Ser Phe Met Arg Thr Phe Ser Ser Ser Gly Asn Ile Phe Trp Pro Val
235                 240                 245                 250

caa aac gtt gta cga agc gtt gaa ggt ggc tgg tta aat aaa act gtg 2079
Gln Asn Val Val Arg Ser Val Glu Gly Gly Trp Leu Asn Lys Thr Val
                255                 260                 265

tct ggc gtt tct gta ggc caa act gga ggt gca tta tgt att gaa tta 2127
Ser Gly Val Ser Val Gly Gln Thr Gly Gly Ala Leu Cys Ile Glu Leu
            270                 275                 280

ggt cat ggc gtc atg att tca cgt gaa tgc ttt gaa gat att gga caa 2175
Gly His Gly Val Met Ile Ser Arg Glu Cys Phe Glu Asp Ile Gly Gln
        285                 290                 295

ata ccc tat gcg gtg gca gaa gac ctt tgt act tct att gaa gct aca 2223
Ile Pro Tyr Ala Val Ala Glu Asp Leu Cys Thr Ser Ile Glu Ala Thr
    300                 305                 310

cta aaa ggc tgg aac att aaa ttt gct tca caa att tac ggt aat gaa 2271
Leu Lys Gly Trp Asn Ile Lys Phe Ala Ser Gln Ile Tyr Gly Asn Glu
315                 320                 325                 330

gcg ttt cct gtt aat atg gca gca tta atg att aga tct agt aag ttt 2319
Ala Phe Pro Val Asn Met Ala Ala Leu Met Ile Arg Ser Ser Lys Phe
                335                 340                 345

tgt tct gca aat ttt gaa ttt ttt aaa aaa tat tcg gcg aga atc atc 2367
Cys Ser Ala Asn Phe Glu Phe Phe Lys Lys Tyr Ser Ala Arg Ile Ile
            350                 355                 360

aag tca aag acc ata agt ctc tat caa aaa atc gac ttg ttt tgt ttt 2415
Lys Ser Lys Thr Ile Ser Leu Tyr Gln Lys Ile Asp Leu Phe Cys Phe
        365                 370                 375

acc cta tca gtt cca ata agt gct ttt caa tat att agc tta gtt att 2463
Thr Leu Ser Val Pro Ile Ser Ala Phe Gln Tyr Ile Ser Leu Val Ile
    380                 385                 390

act agt ata att tgt cca gtg ttg cac att cca cta gta aca caa tta 2511
Thr Ser Ile Ile Cys Pro Val Leu His Ile Pro Leu Val Thr Gln Leu
395                 400                 405                 410

ttt atg tta tta cca acg tta gtc tgt tac ttt agt caa agt ttg gtc 2559
Phe Met Leu Leu Pro Thr Leu Val Cys Tyr Phe Ser Gln Ser Leu Val
                415                 420                 425

gat act gtc ttt cat ttg aca aac ggt atg aaa ttc tta gat tta ttg 2607
Asp Thr Val Phe His Leu Thr Asn Gly Met Lys Phe Leu Asp Leu Leu
            430                 435                 440

att tat gaa gta gaa tca atg ttg tta tat ggg tct ttt tat ttt att 2655
Ile Tyr Glu Val Glu Ser Met Leu Leu Tyr Gly Ser Phe Tyr Phe Ile

445                 450                 455

aca atc aag tct acc gta cta gct tta atg aac aaa cct gct aaa ttc      2703
Thr Ile Lys Ser Thr Val Leu Ala Leu Met Asn Lys Pro Ala Lys Phe
    460                 465                 470

ata gtt aca ccg aag gtt aat gag cat ata act ttt ctg cat gca ata      2751
Ile Val Thr Pro Lys Val Asn Glu His Ile Thr Phe Leu His Ala Ile
475                 480                 485                 490

aga aat cat tat caa gga atc tta ttt tca ata ttt aca ata att gca      2799
Arg Asn His Tyr Gln Gly Ile Leu Phe Ser Ile Phe Thr Ile Ile Ala
                495                 500                 505

tgt atc gca att tct gga agt tat tgg gta tta tta tca ttt att ccg      2847
Cys Ile Ala Ile Ser Gly Ser Tyr Trp Val Leu Leu Ser Phe Ile Pro
            510                 515                 520

ggt tgt ttt ggg ttt ttg ttc gaa atg caa gct aat cat cgg aca tca      2895
Gly Cys Phe Gly Phe Leu Phe Glu Met Gln Ala Asn His Arg Thr Ser
        525                 530                 535

gaa gaa caa ata aaa gcg gat aaa tta cag agt tac aac aat aag gca      2943
Glu Glu Gln Ile Lys Ala Asp Lys Leu Gln Ser Tyr Asn Asn Lys Ala
    540                 545                 550

tta caa tct ggc aac acg gaa aca gtt gat tgg aat gat taaaattaca      2992
Leu Gln Ser Gly Asn Thr Glu Thr Val Asp Trp Asn Asp
555                 560                 565

tcattactat tttttagtat aggagtaaaa aaggtgagaa gtcagaaatt ttaatctggg      3052

taaagtagtg aattgtttta tcgaatgata aactgctgaa tctattttat aacgaaggac      3112

acagcgcttt aatccgtatg caaaaagatg gctgaaaagg aaactcggct taaacagcat      3172

aaatgtcagt actgttttta cggatcaccg ggagtcagcg gttcgacttt taccagcatc      3232

cgctgttaca actgcagact ggcaccctaa agttgagtta ttgtatacga cctataatga      3292

ttttatacct tatgcactag ctcaatgttt aaaacagaca tatgataaca cgcagggcgt      3352

<210>  6
<211>  567
<212>  PRT
<213>  Pediococcus damnosus

<400>  6

Met Leu Asn Asp Asn Asp Ser Glu Leu Lys Lys Phe His Leu Phe His
1               5                   10                  15

Ser Lys Pro Val Phe Val Pro Val Ile Leu Ile Ile Trp Leu Phe Ile
            20                  25                  30

Met Cys Leu Tyr Glu Tyr Leu Thr Tyr Thr Asp Ser Ile Leu Pro Ile
        35                  40                  45

Leu Ala Lys Lys Gln Pro Leu Glu Val Ile Leu Pro Ile Phe Asn Gln
    50                  55                  60

Leu Phe Val Ala Leu Phe Phe Leu Leu Gly Ile Thr Asn Ile Ile Ile
65                  70                  75                  80

Ala Ile Arg Tyr Ala Met Ile Lys Asp Lys Ala Lys Glu Ser Glu Leu
                85                  90                  95

Ala Ile Leu Ala Lys Glu Thr Pro Ala Asp Trp His Pro Lys Val Glu
            100                 105                 110

Leu Leu Tyr Thr Thr Tyr Asn Asp Phe Ile Pro Tyr Ala Leu Ala Gln
        115                 120                 125

Cys Leu Lys Gln Thr Tyr Asp Asn Thr Gln Gly Val Ile Leu Asp Asn
    130                 135                 140

Ser Thr Asp Pro Lys Tyr Ile Lys Met Ile Asp Asp Phe Val Ile Ala
145                 150                 155                 160

His Pro Asn Val Lys Leu Val Arg Asp Ser Gln Asn Lys His Ala Lys
                165                 170                 175

Ala Gly Asn Leu Asn Asn Tyr Leu Cys Asn Gly Thr His Asp Tyr Asp
            180                 185                 190

Tyr Phe Val Ile Leu Asp Ser Asp Glu Leu Leu Glu Asn Arg Phe Val
        195                 200                 205

Glu Lys Cys Leu Lys Met Phe Tyr Tyr Asn Asp Ile Gly Ile Leu Gln
    210                 215                 220

Cys Asn His Ile Ser Gly Gln Asn His Asn Ser Phe Met Arg Thr Phe
225                 230                 235                 240

Ser Ser Ser Gly Asn Ile Phe Trp Pro Val Gln Asn Val Val Arg Ser
                245                 250                 255

Val Glu Gly Gly Trp Leu Asn Lys Thr Val Ser Gly Val Ser Val Gly
            260                 265                 270

Gln Thr Gly Gly Ala Leu Cys Ile Glu Leu Gly His Gly Val Met Ile
        275                 280                 285

Ser Arg Glu Cys Phe Glu Asp Ile Gly Gln Ile Pro Tyr Ala Val Ala
    290                 295                 300

Glu Asp Leu Cys Thr Ser Ile Glu Ala Thr Leu Lys Gly Trp Asn Ile
305                 310                 315                 320

Lys Phe Ala Ser Gln Ile Tyr Gly Asn Glu Ala Phe Pro Val Asn Met
                325                 330                 335

Ala Ala Leu Met Ile Arg Ser Ser Lys Phe Cys Ser Ala Asn Phe Glu
            340                 345                 350

Phe Phe Lys Lys Tyr Ser Ala Arg Ile Ile Lys Ser Lys Thr Ile Ser
        355                 360                 365

Leu Tyr Gln Lys Ile Asp Leu Phe Cys Phe Thr Leu Ser Val Pro Ile
    370                 375                 380

Ser Ala Phe Gln Tyr Ile Ser Leu Val Ile Thr Ser Ile Ile Cys Pro
385                 390                 395                 400

Val Leu His Ile Pro Leu Val Thr Gln Leu Phe Met Leu Leu Pro Thr
                405                 410                 415

Leu Val Cys Tyr Phe Ser Gln Ser Leu Val Asp Thr Val Phe His Leu
            420                 425                 430

Thr Asn Gly Met Lys Phe Leu Asp Leu Leu Ile Tyr Glu Val Glu Ser
        435                 440                 445

Met Leu Leu Tyr Gly Ser Phe Tyr Phe Ile Thr Ile Lys Ser Thr Val
    450                 455                 460

Leu Ala Leu Met Asn Lys Pro Ala Lys Phe Ile Val Thr Pro Lys Val
465                 470                 475                 480

Asn Glu His Ile Thr Phe Leu His Ala Ile Arg Asn His Tyr Gln Gly
                485                 490                 495

Ile Leu Phe Ser Ile Phe Thr Ile Ile Ala Cys Ile Ala Ile Ser Gly
            500                 505                 510

Ser Tyr Trp Val Leu Leu Ser Phe Ile Pro Gly Cys Phe Gly Phe Leu
        515                 520                 525

Phe Glu Met Gln Ala Asn His Arg Thr Ser Glu Glu Gln Ile Lys Ala
    530                 535                 540

Asp Lys Leu Gln Ser Tyr Asn Asn Lys Ala Leu Gln Ser Gly Asn Thr
545                 550                 555                 560

Glu Thr Val Asp Trp Asn Asp
                565

<210>  7
<211>  24
<212>  DNA
<213>  Artificial sequence

<220>
<223>  Oligo V

<400>  7
tctagaaatt aaaggactgt gtaa                                              24

<210>  8
<211>  26
<212>  DNA
<213>  Artificial sequence

<220>
<223>  Oligo VI

<400>  8
tctagattaa tcattccaat caactg                                            26

<210>  9

<211>  1701
<212>  DNA
<213>  Pediococcus damnosus

<400>  9
atgttaaatg ataatgattc agaactaaaa aaatttcact tgtttcattc taaaccagtc      60

tttgtaccag ttattttaat tatttggttg tttattatgt gcttatatga atatttaaca     120

tacacagata gcatacttcc tattttagct aaaaagcaac cactagaagt aattttacct     180

atattcaatc aattatttgt ggcacttttc tttttgcttg gaataactaa tattattatc     240

gctatccgct atgcaatgat taaagacaaa gcaaaagaat ccgaactagc aatactcgca     300

aaagaaacgc ctgcagactg gcaccctaaa gttgagttat tgtatacgac ctataatgat     360

tttatacctt atgcactagc tcaatgttta aaacagacat atgataacac gcagggcgtt     420

attttggata actctacaga ccccaaatac atcaagatga ttgatgattt tgtgatagcc     480

catcctaatg taaagttagt cagagattct caaaacaagc atgctaaagc tggaaactta 540
aacaattatt tgtgtaatgg cactcatgac tacgattact ttgttatcct agatagcgat 600
gaattattag aaaatagatt tgtagaaaaa tgtttaaaga tgttttatta caatgatatt 660
ggcattcttc agtgtaatca cattagtgga caaaaccaca attcgtttat gcgtactttc 720
tctagttctg gcaatatttt ttggccagtg caaaacgttg tacgaagcgt tgaaggtggc 780
tggttaaata aaactgtgtc tggcgtttct gtaggccaaa ctggaggtgc attatgtatt 840
gaattaggtc atggcgtcat gatttcacgt gaatgctttg aagatattgg acaaataccc 900
tatgcggtgg cagaagacct ttgtacttct attgaagcta cactaaaagg ctggaacatt 960
aaatttgctt cacaaattta cggtaatgaa gcgtttcctg ttaatatggc agcattaatg 1020
attagatcta gtaagttttg ttctgcaaat tttgaatttt ttaaaaaata ttcggcgaga 1080
atcatcaagt caaagaccat aagtctctat caaaaaatcg acttgttttg ttttacccta 1140
tcagttccaa taagtgcttt tcaatatatt agcttagtta ttactagtat aatttgtcca 1200
gtgttgcaca ttccactagt aacacaatta tttatgttat taccaacgtt agtctgttac 1260
tttagtcaaa gtttggtcga tactgtcttt catttgacaa acggtatgaa attcttagat 1320
ttattgattt atgaagtaga atcaatgttg ttatatgggt ctttttattt tattacaatc 1380
aagtctaccg tactagcttt aatgaacaaa cctgctaaat tcatagttac accgaaggtt 1440
aatgagcata taacttttct gcatgcaata agaaatcatt atcaaggaat cttattttca 1500
atatttacaa taattgcatg tatcgcaatt tctggaagtt attgggtatt attatcattt 1560
attccgggtt gttttgggtt tttgttcgaa atgcaagcta atcatcggac atcagaagaa 1620
caaataaaag cggataaatt acagagttac aacaataagg cattacaatc tggcaacacg 1680
gaaacagttg attggaatga t 1701